# EUROPEAN PATENT SPECIFICATION

(11) **EP 2 971 191 B1**
(45) Date of publication and mention of the grant of the patent: **11.12.2019**
(21) Application number: 14764509.7
(22) Date of filing: 14.03.2014
(51) Int. Cl.: C13K 5/00, A23C 23/00, C07H 1/08, A23C 9/142, C07H 3/04

(54) **LACTOSE RECOVERY**
LACTOSERÜCKGEWINNUNG
RÉCUPÉRATION DE LACTOSE

(30) Priority: 15.03.2013 US 201361794853 P
(43) Date of publication of application: 20.01.2016
(73) Proprietor: Fonterra Co-Operative Group Limited, Auckland (NZ); Aurecon New Zealand Limited, Wellington (NZ)
(72) Inventor: STEPHENSON, Paul, Wellington (NZ); STEVENS, Geoffrey Shaun, Palmerston North (NZ); BERRIDGE, Ian Stuart, Palmerston North (NZ)
(74) Representative: Walker, Ross Thomson
(86) International application number: PCT/IB2014/059773
(87) International publication number: WO 2014/141164

(56) References cited:
- EP-A1- 1 869 984
- WO-A1-00/12200
- WO-A1-02/50089
- WO-A1-2012/047122
- AU-B2- 2002 218 862
- CN-B- 101 491 287
- US-A- 3 166 486
- US-A- 4 955 363

## Description

### FIELD OF THE INVENTION

The present invention relates to a method of recovering a lactose-containing extract from one or more lactose-containing liquid streams.

### BACKGROUND TO THE INVENTION

During lactose crystallisation, lactose is typically lost in one or more waste streams, reducing the lactose yield. EP 1869984 A1 discloses a process for obtaining lactose from whey, by extracting protein from whey by ultrafiltration, subjecting the ultrafiltration permeate to a membrane filtration, electrodialysis or ion exchange process to remove water, concentrating the lactose concentrate to at least 60% dry matter by evaporation and crystallize the lactose from the concentrate. A part from the permeate is recirculated to the continuous process, before the step of evaporation.

It is an object of the present invention to provide a method of obtaining lactose from one or more lactose-containing liquid streams or which at least provides the public with a useful choice.

### SUMMARY OF THE INVENTION

In one aspect the invention relates to a method of obtaining lactose from a lactose-containing liquid source in accordance to claim 1.

In one aspect the invention relates to a method of obtaining lactose from a lactose-containing liquid source comprising
subjecting a lactose-containing liquid source to a lactose crystallisation process to generate one or more lactose-containing liquid streams,
the one or more lactose-containing liquid streams being processed by heating and/or pH adjustment,
subjecting the one or more lactose-containing liquid streams to one or more processes to generate a lactose-containing extract, the one or more processes comprising
i) a first step, to remove one or more of cells, proteins, polypeptides, polysaccharides, lipid, ions or salts, comprising
   a) centrifugal separation to produce a light phase and a heavy phase with optional ultrafiltration or nanofiltration of the light phase, or
   b) ultrafiltration to generate an ultrafiltration permeate and an ultrafiltration retentate, and
ii) a second step comprising nanofiltration of
   a) the light phase from the centrifugal separator, or
   b) the permeate from ultrafiltration, or
   c) the retentate from nanofiltration,
adding the lactose-containing extract to the lactose-containing liquid source before, during or after entry into the crystallisation process.

In one aspect the invention relates to a method of obtaining lactose from a lactose-containing liquid source comprising
subjecting a lactose-containing liquid source to a lactose crystallisation process to generate one or more lactose-containing liquid streams,
the one or more lactose-containing liquid streams being processed by heating and/or pH adjustment,
subjecting the one or more lactose-containing liquid streams to one or more processes to generate a lactose-containing extract, the one or more processes comprising
i) a first step, to remove one or more of cells, proteins, polypeptides, polysaccharides, lipid, ions or salts, comprising
   a) centrifugal separation to produce a light phase and a heavy phase with optional ultrafiltration or nanofiltration of the light phase to generate a permeate or a retentate, or
   b) ultrafiltration to generate an ultrafiltration permeate and an ultrafiltration retentate, and
ii) a second step comprising nanofiltration of
   a) the light phase from the centrifugal separator, or
   b) the permeate from optional filtration, when the optional filtration is carried out with a membrane having a molecular weight cutoff greater than about 1,000 Daltons to about 10,000 Daltons, or
   c) the retentate from optional filtration, when the optional filtration is carried out with a membrane having a molecular weight cutoff less than about 100 Daltons to about 1,000 Daltons, or
   d) the ultrafiltration permeate,
adding the lactose-containing extract to the lactose-containing liquid source before, during or after entry into the crystallisation process.

The following embodiments may relate to any of the above aspects.

In some embodiments the one or more processes comprise centrifugal separation in combination with
i) ultrafiltration,
ii) nanofiltration, or
iii) ultrafiltration and nanofiltration.

In some embodiments the one or more processes comprise ultrafiltration in combination with
i) nanofiltration,
ii) centrifugal separation, or
iii) nanofiltration and centrifugal separation.

In some embodiments the one or more processes comprise nanofiltration in combination with
i) ultrafiltration,
ii) centrifugal separation, or
iii) ultrafiltration and centrifugal separation.

In one embodiment the one or more processes comprises centrifugal separation and nanofiltration.

In one embodiment the one or more processes comprises centrifugal separation, ultrafiltration and nanofiltration.

In one embodiment the one or more processes comprises ultrafiltration and nanofiltration.

In one embodiment the one or more processes comprises two or more nanofiltration steps.

In one embodiment the method comprises subjecting the light phase from centrifugal separation to
ultrafiltration and collecting the permeate,
nanofiltration and collecting the retentate, or
ultrafiltration, and subjecting the ultrafiltration permeate to nanofiltration and collecting the retentate.

In some embodiments the filtration comprises use of a membrane or membranes with a molecular weight cutoff
i) greater than about 1,000 Daltons to about 10,000 Daltons,
ii) less than about 100 Daltons to about 1,000 Daltons, or
iii) both (i) and (ii).

In some embodiments the filtration comprises use of a membrane or membranes having a pore size of
i) greater than about 1 nm to about 5 nm,
ii) less than about 0.1 nm to about 0.5 nm, or
iii) both (i) and (ii).

In one embodiment the nanofiltration comprises two or more nanofiltration steps.

In some embodiments the lactose-containing extract is
(a) added to a liquid composition comprising lactose, casein or whey, or any combination of any two or more thereof,
(b) used to standardise a dairy product,
(c) used in an ethanol production process,
(d) added to the lactose-containing liquid source before, during or after entry into the crystallisation process, or
(e) any combination of any two or more of (a) to (d).

In one embodiment the method comprises subjecting the lactose-containing liquid source to evaporation before crystallisation.

In one embodiment a majority of the lactose-containing extract is added to the lactose-containing liquid.

In one embodiment substantially all of the lactose-containing extract is added to the lactose-containing liquid.

In one embodiment at least 60, 65, 70, 75, 80, 85, 90, 95 or 99% of the lactose-containing extract is added to the lactose-containing liquid, and useful ranges may be selected between any of these values.

In one embodiment the heavy phase is sent to waste.

In one embodiments the heavy phase is recycled for further processing by the centrifugal separator.

In one embodiment the method does not include a chromatographic separation step.

In some embodiments the heating, pH adjustment, or heating and pH adjustment reduces the solubility of calcium in the lactose-containing liquid stream.

The heating, pH adjustment, or heating and pH adjustment precipitates calcium from the lactose-containing liquid stream.

In one embodiment the lactose-containing liquid stream is heated to a temperature of 70° C to 100° C.

In one embodiment the lactose-containing liquid stream is heated to a temperature of about 75° C to about 85° C.

In one embodiment the lactose-containing liquid stream is heated to a temperature of about 75° C.

In one embodiment the lactose-containing liquid stream is heated at the temperature for 30 seconds, 1, 2, 3, 4, 5, 6, 7, 8, 9, 10, 11, 12, 13, 14, 15, 16, 17, 18, 19 or 20 minutes, and useful ranges may be selected between any of these values.

In one embodiment the pH of the lactose-containing liquid stream is adjusted to a pH of 6.7 to 8.0. In one embodiment the pH of the lactose-containing liquid stream is maintained at a pH of 6.7 to 8.0.

In one embodiment the pH of the lactose-containing liquid stream is adjusted to a pH of about 6.9 to about 7.4. In one embodiment the pH of the lactose-containing liquid stream is maintained at a pH of about 6.9 to about 7.4.

In one embodiment the pH of the lactose-containing liquid stream is adjusted to a pH of about 7.0 to about 7.2. In one embodiment the pH of the lactose-containing liquid stream is maintained at a pH of about 7.0 to about 7.2.

In some embodiments the lactose-containing liquid stream has
a temperature of about 70° C to about 100° C and a pH of about 6.7 to about 8.0,
a temperature of about 70° C to about 100° C and a pH of about 6.9 to about 7.4,
a temperature of about 70° C to about 100° C and a pH of about 7.0 to about 7.2,
a temperature of about 75° C to about 85° C and a pH of about 6.7 to about 8.0,
a temperature of about 75° C to about 85° C and a pH of about 6.9 to about 7.4, or
a temperature of about 75° C to about 85° C and a pH of about 7.0 to about 7.2.

In one embodiment the lactose-containing liquid stream is heated and then pH adjusted before centrifugal separation or filtration.

In one embodiment the lactose-containing liquid stream is pH adjusted and then heated before centrifugal separation or filtration.

In one embodiment the pH is adjusted by adding a pH adjuster to the inlet of the centrifugal separator, or membrane filter.

In one embodiment the pH is adjusted by adding sodium hydroxide (NaOH) or potassium hydroxide (KOH).

In one embodiment an amount of the light phase from the centrifugal separator is added to the one or more lactose-containing liquid streams feeding the centrifugal separator.

In one embodiment the recycled fraction comprises about 40% to about 100% of the liquid volumetric flow into the centrifugal separator.

In one embodiment the lactose-containing liquid stream is diluted, such as with water, milk permeate, whey permeate or any combination of any two or more thereof, before being subjected to centrifugal separation or membrane filtration.

In one embodiment the calcium concentration of the lactose-containing liquid stream is about or is adjusted to about 0.2% by weight to about 0.7% by weight before being subjected to centrifugal separation or membrane filtration.

In some embodiments the lactose-containing liquid stream is selected from
i) mother liquor,
ii) wash water, or
iii) mother liquor and wash water.

In one embodiment the centrifugal separator is a clarifier or a decanter.

In some embodiments the centrifugal separation, ultrafiltration, or centrifugal separation and ultrafiltration, removes at least 60, 65, 70, 75, 80, 85, 90, 95% by weight of the insoluble solids from the lactose-containing liquid stream that has been subjected to
i) heat,
ii) pH adjustment, or
iii) heat and pH adjustment.

In one embodiment the ultrafiltration membrane is selected from a spiral membrane, ceramic membrane, vibratory shear enhanced membrane, hollow fibre membrane, or a flat sheet membrane.

In one embodiment a high-temperature tolerant ultrafiltration membrane is used.

In one embodiment the ultrafiltration membrane is a ceramic membrane.

In one embodiment the lactose-containing liquid stream is diluted with water at a ratio of between about 1:2 to about 1:0.5. Preferably the lactose-containing liquid stream is diluted in water at a ratio of about 1:1 of mother liquor to water.

In one embodiment the molecular weight cut-off (MWCO) of the ultrafiltration membrane is 1,000 Daltons to 10,000 Daltons.

In one embodiment the temperature of the lactose-containing stream subjected to ultrafiltration (i.e. the ultrafiltration feed stream) is up to 80° C to 90° C.

In one embodiment the ultrafiltration retentate has a total solids content of about 20% by weight to about 35% by weight.

In one embodiment the filtration comprises diafiltration (DF), such as with water. DF is used to increase the recovery of lactose in the ultrafiltration permeate.

In one embodiment the nanofiltration membrane is selected from a spiral membrane, ceramic membrane, hollow fibre membrane, or a flat sheet membrane.

In some embodiments, a polymer membrane is used for ultrafiltration and the permeate flux during ultrafiltration is about 4, 5, 6, 7, 8, 9, 10, 11, 12, 13, 14, 15, 16, 17, 18, 19 or 20 litres per hour per m² (LMH), and useful ranges may be selected between any of these values.

In some embodiment a high-temperature tolerant nanofiltration membrane is used.

In one embodiment the nanofiltration membrane is a ceramic membrane.

In some embodiments, a high-temperature tolerant UF membrane is used for ultrafiltration and the permeate flux during ultrafiltration is at least 40, 45, 50, 55, 60, 65, 70, 75, 80, 85, 90, 95, 100, 105, 110, 115, 120 litres per hour per m² (LMH), and useful ranges may be selected between any of these values.

In one embodiment the temperature of the lactose-containing stream subjected to nanofiltration (i.e. the nanofiltration feed stream) is 45° C to 90° C.

In one embodiment the nanofiltration retentate has a total solids content of about 20% by weight to about 30% by weight.

In one embodiment the nanofiltration further comprises diafiltration, such as with water. DF is used to increase the removal of ash into the nanofiltration permeate.

In one embodiment the diafiltration is carried out with hydrochloric acid (HCI).

In one embodiment the concentration of HCI is 0.0625% by weight.

In one embodiment the pH of the nanofiltration feed stream is adjusted to 5.8 to 6.2.

In one embodiment concentrated HCl is used to adjust the pH of the nanofiltration feed stream.

In one embodiment diluted HCl is used to adjust the pH of the nanofiltration feed stream.

In one embodiment about 10% to about 20% by weight dilute HCl is added to the NF feed stream.

In one embodiment the HCl is injected in-line.

In one embodiment the lactose-containing extract comprises at least 70% by weight to 85% by weight lactose on a dry basis.

In one embodiment the lactose-containing extract comprises at least about 20% by weight to about 30% by weight total solids.

In one embodiment the lactose-containing extract is added to the lactose-containing liquid source at a rate of about 10, 12, 14, 16, 18, 20, 22, 24, 26, 28, 30% of lactose source volumetric flow, and useful ranges may be selected between any of these values.

In one embodiment the inventive method results in a lactose yield from the lactose-containing liquid source of at least 70% to 95%.

In one embodiment the lactose-containing extract has a higher lactose concentration by weight of total solids than the lactose-containing source.

In some embodiments the method is run as a continuous process.

In some embodiments the method is run on a continuous process basis while controlling the amount of the solids non-lactose retuned to the crystalliser in the lactose-containing extract.

In some embodiments the lactose-containing extract has a non-lactose solids/total solids (SNL/TS) ratio of less than 0.15, 0.16, 0.17, 0.18, 0.19, 0.20, 0.21, 0.22, 0.23, 0.24, 0.25, 0.26, 0.27, 0.28, 0.29 or 0.30.

In one embodiment the lactose concentration, by weight of the total solids, in the lactose-containing extract is 8, 10, 12, 14, 16, 18, 20, 22, 24, 26, 28, 30, 32, 34, 36, 38, 40, 42, 44 or 46% higher than in the lactose-containing liquid stream, and useful ranges may be selected between any of these values.

In one embodiment the lactose concentration, by weight of the total solids, in the lactose-containing extract is at least 70, 75, 80, 85, 90, 95, 100, 105, 110% of the lactose-containing liquid source, and useful ranges may be selected between any of these values.

The term "comprising" as used in this specification and claims means "consisting at least in part of". When interpreting statements in this specification and claims which include the term "comprising", other features besides the features prefaced by this term in each statement can also be present. Related terms such as "comprise" and "comprised" are to be interpreted in similar manner.

As used herein the term "and/or" means "and" or "or", or both.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

To those skilled in the art to which the invention relates, many changes in construction and differing embodiments and applications of the invention will suggest themselves without departing from the scope of the invention as defined in the appended claims. The disclosures and the descriptions herein are purely illustrative and are not intended to be in any sense limiting.

### BRIEF DESCRIPTION OF THE DRAWINGS

The invention will now be described by way of example only and with reference to the drawings in which:

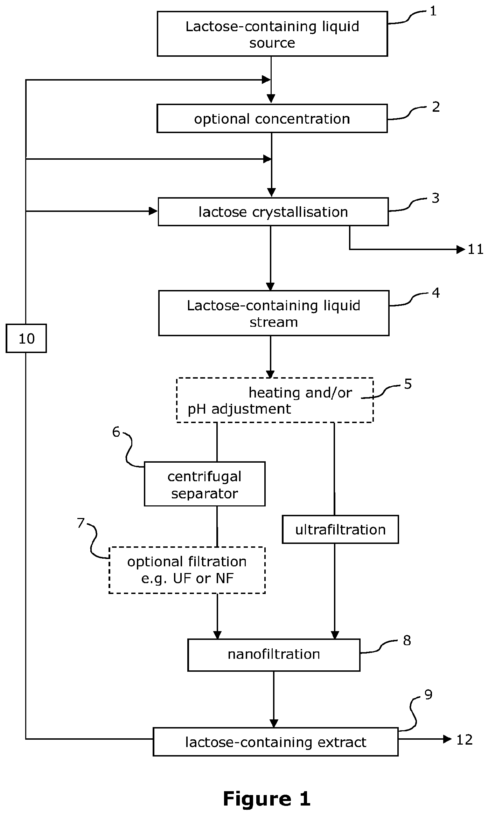

Figure 1 shows a process flow diagram of the process of the present invention.

### DETAILED DESCRIPTION OF THE INVENTION

The invention relates to a method of recovering lactose from a lactose-containing liquid source, such as milk or a milk derivative. The method generally includes subjecting a lactose-containing liquid source to a lactose crystallisation process to generate one or more lactose-containing liquid streams, subjecting the one or more lactose-containing liquid streams to (i) heat, (ii) pH adjustment, or (iii) heat and pH adjustment and processing that lactose stream to generate a lactose-containing extract. The processing includes
i) a first step, to remove one or more of cells, proteins, polypeptides, polysaccharides, lipid, ions or salts, comprising
   a) centrifugal separation to produce a light phase and a heavy phase with optional filtration of the light phase, or
   b) ultrafiltration to generate an ultrafiltration permeate and an ultrafiltration retentate, and
ii) a second step comprising nanofiltration.

The lactose-containing extract may be
(a) added to a liquid composition comprising lactose, casein or whey, or any combination of any two or more thereof,
(b) used to standardise a dairy product,
(c) used in an ethanol production process,
(d) added to the lactose-containing liquid source before, during or after entry into the crystallisation process, or
(e) any combination of any one or more of (a) to (d).

The invention further relates to a method of increasing the lactose yield from the lactose-containing liquid source.

The inventors have determined that lactose can be recovered from the lactose-containing liquid source and returned to the crystalliser on a continuous basis while controlling the amount of the solids non-lactose retuned to the crystalliser.

### 1. Lactose-containing liquid source

The lactose-containing liquid source in the process is generally obtained from mammalian milk. Any fraction or milk derivative can be utilised in the invention provided it contains lactose.

In one embodiment, the lactose-containing liquid source may be derived from any mammalian milk including but not limited to bovine, sheep, goat, pig, mouse, water buffalo, camel, yak, horse, donkey, llama or human milk with bovine milk being a preferred source.

In one embodiment the mammalian milk source is processed to remove protein and fat prior to being used in the inventive process.

By way of example, the lactose-containing liquid source may be generated from upstream milk processes, such as cheese manufacture, or casein manufacture, whey protein concentrate manufacture or milk protein concentrate manufacture that produce whey, whey permeate or milk permeate.

The lactose-containing source, such as whey, whey permeate or milk permeate from upstream dairy processing, may be processed by one or more filtration steps and may include one or more concentration steps.

In one embodiment the lactose-containing liquid source is the permeate resulting from filtration of whole milk, recombined, powdered or fresh skim milk, recombined or reconstituted whole or skim milk powder, skim milk concentrate, skim milk retentate, concentrated milk, ultrafiltered milk retentate, milk protein concentrate (MPC), milk protein isolate (MPI), calcium depleted milk protein concentrate (MPC), low fat milk, low fat milk protein concentrate (MPC), casein, caseinate, colostrum, a colostrum fraction, colostrum protein concentrate (CPC), colostrum whey, whey (including sweet whey, lactic acid whey, mineral acid whey, or reconstituted whey powder), whey protein isolate (WPI), whey protein concentrate (WPC), a composition derived from any milk or colostrum processing stream, a composition derived from the retentate or permeate obtained by ultrafiltration or microfiltration of any milk or colostrum processing stream, or any combination of any two or more thereof. It should be understood that the source of these derivatives may be milk or colostrum or a combination thereof.

The ratio of non-lactose solids (SNL) to total solids (TS) in a typical cheese whey permeate stream is about 0.17. i.e. 17 %w/w of the solids in the feed stream consist of non-lactose solids and 83% w/w of the solids is lactose. The SNL includes ash, protein, non-protein nitrogen, fat and any organic material.

In one embodiment the permeate is concentrated by removing water. Any concentration process can be used to effect this, such as by reverse osmosis or evaporation. Both reverse osmosis and evaporation may be used.

For example, in some embodiments the permeate is concentrated by reverse osmosis.

In one embodiment the permeate after concentration by reverse osmosis comprises at least about 10% to about 30% by weight total solids and useful ranges may be selected between any of these values.

In one embodiment the permeate is additionally or alternatively concentrated by evaporation. The removal of water is limited by the need to be able to use temperature to control supersaturation and the need to maintain the permeate in a liquid state that can be pumped and separated from the crystals during crystallisation. Evaporation for the purpose of concentration can be carried out by any evaporative process known, such as the use of evaporators such as a falling film evaporator, rising film evaporator, plate evaporator, or multiple effect evaporator.

In one embodiment the permeate after concentration by evaporation comprises at least about 40, 45, 50, 55, 60, 65, 70, 75 or 78% by weight total solids and useful ranges may be selected between any of these values (for example, from about 40 to about 78, about 40 to about 70, about 40 to about 60, about 40 to about 50, about 45 to about 78, about 45 to about 70, about 45 to about 55, about 55 to about 78, about 55 to about 70, about 55 to about 60, about 60 to about 78, about 60 to about 70, about 70 to about 78, about 70 to about 75% by weight total solids).

In some embodiments the lactose-containing liquid source comprises precipitated calcium salts.

### 2. Lactose-containing liquid stream

The lactose-containing liquid stream is generated from processing the lactose-containing liquid source by a crystallisation process.

The crystallisation process produces a magma that contains the solid lactose crystals and a liquor. The lactose crystals are separated from the magma by centrifugation and are typically washed, to produce a wash water, and the crystals are then dried and further processed to make a lactose product.

The liquor generally contains small lactose crystals, dissolved lactose, and impurities such as cells, proteins, polypeptides, polysaccharides, lipid, ions or salts. The liquor is known as mother liquor.

In some embodiments where the lactose-containing liquid source has been concentrated to a high solids content (for example, above about 65 to about 70% TS), the mother liquor may also contain precipitated calcium salts.

The SNL/TS ratio in mother liquor from lactose processing is typically in the range 0.40 to 0.60. i.e. 40 to 60% w/w of the solids in the mother liquor are non-lactose solids and 40 to 60% w/w are lactose.

In one embodiment the mother liquor is fed into the process of the present invention either directly from the mother liquor out-flow from an existing lactose process or from mother liquor storage silos.

In one embodiment the wash water containing small lactose crystals and/or dissolved lactose is fed into the process of the present invention.

In one embodiment the lactose-containing liquid stream is diluted, such as with water, milk permeate, whey permeate or any combination of any two or more thereof, before being subjected to centrifugal separation or membrane filtration. Milk permeate is a by-product of ultrafiltration of milk to extract protein. Whey permeate is a by-product of the ultrafiltration of cheese or casein whey to extract protein

### 3. Processing methods

The processing method for producing the lactose-containing extract is explained below and is shown in Figure 1.

Briefly stated, a lactose-containing liquid source 1 is prepared for crystallisation 3, which may include optional concentration steps 2 prior to crystallisation 3 (i.e. filtration and/or evaporation). The lactose-containing liquid source 1 is subjected to crystallisation 3 in which lactose in the lactose-containing liquid source 1 is crystallised. Methods of lactose crystallisation are known in the art, for example, see "Crystallization" 4th ed by J W Mullin. Lactose can be crystallised out of solution, and is usually accelerated by the addition of fine lactose "seeding" crystals. Typically lactose crystallisation is carried out by first concentrating the lactose in the solution to become super saturated followed by controlled cooling and inducing crystal growth in a cooling crystalliser. The crystallisation process produces a magma which contains lactose crystals 11 and a liquor. Crystals 11 are extracted from the magma by centrifugation to yield raw lactose and a lactose-containing liquid stream 4 (i.e. mother liquor). This raw lactose is then generally washed, recovered by centrifugation and dried to give high purity lactose. The wash water typically contains lactose.

Mother liquor typically contains about 17% to 21% lactose by weight of total solids. The mother liquor also typically contains about 32% by weight total solids (TS).

The lactose-containing liquid stream 4, such as the wash water and mother liquor (i.e. the liquor phase), may contain quantities of cells, proteins, polypeptides, polysaccharides, lipid, ions, salts and lactose. The mother liquor is typically sold as a cattle feed supplement.

In some embodiments the lactose-containing liquid streams 4 also contain precipitated salts, such as calcium salts. The lactose-containing liquid stream can contain precipitated salts when the lactose-containing liquid source is concentrated to a high total solids prior to crystallisation.

One or more of the lactose-containing liquid streams are further processed and include a heating step and/or pH adjustment step 5 prior to the further processing. The further processing of the lactose-containing liquid streams includes
▪ centrifugal separation 6 in combination with filtration 7 (e.g. nanofiltration and/or ultrafiltration, followed by nanofiltration 8), or
▪ ultrafiltration followed by nanofiltration 8.

The further processing generates a lactose-containing extract 9 that may be
recycled 10 to crystallisation 3, or
used as a feed material 12 in other processes such as the standardisation of a milk product or ethanol production.

In one embodiment the lactose-containing extract is recovered from the one or more lactose-containing liquid streams by obtaining the light phase from centrifugal separation and subsequently
obtaining the permeate from ultrafiltration of the light phase,
obtaining the retentate from nanofiltration of the light phase, or
obtaining the permeate from ultrafiltration of the light phase and obtaining the retentate from nanofiltration of the ultrafiltration permeate.

In some embodiments the filtration is carried out by filtering the one or more lactose-containing liquid streams to remove particles from the one or more lactose-containing liquid streams with a molecular weight
i) greater than about 1,000 Daltons to about 10,000 Daltons,
ii) less than about 100 Daltons to about 1,000 Daltons, or
iii) both (i) and (ii).

In some embodiments the filtration is carried out by filtering the one or more lactose-containing liquid streams with a membrane or membranes having a pore size of
i) greater than about 1 nm to about 5 nm,
ii) less than about 0.1 nm to about 0.5 nm, or
iii) both (i) and (ii).

These processes are described below.

### 3.1 Heating and pH adjustment

The lactose-containing liquid stream 4 is heated and/or pH adjusted 5 to precipitate minerals and at least some protein that may be present.

For example, the pH adjustment and/or heat will reduce the solubility of calcium in the lactose-containing liquid stream 4. This leads to precipitation of calcium salts (such as calcium phosphate and calcium sulphate) from the lactose-containing liquid stream 4.

The lactose-containing liquid stream 4 is heated to a sufficient temperature to dissolve substantially all of the lactose in the lactose-containing liquid stream.

In one embodiment the lactose-containing liquid stream 4 is direct heated, for example, by direct steam injection. In alternate embodiments the lactose-containing liquid stream is indirectly heated, for example, by a heat exchanger.

In one embodiment the lactose-containing liquid stream 4 is heated to about 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100° C, and useful ranges may be selected between any of these values (for example, from about 70 to about 90, 70 to about 100, about 70 to about 85, about 70 to about 82, about 70 to about 79, about 70 to about 75, about 72 to about 100, about 72 to about 90, about 72 to about 88, about 72 to about 85, about 72 to about 81, about 72 to about 78, about 72 to about 75, about 75 to about 100, about 75 to about 90, about 75 to about 88, about 75 to about 87, about 75 to about 85, about 75 to about 82, about 75 to about 80, about 78 to about 100, about 78 to about 90, about 78 to about 87, about 78 to about 85, about 78 to about 81, about 80 to about 100, about 80 to about 90, about 80 to about 88, about 80 to about 86, about 80 to about 84, about 84 to about 100, about 84 to about 90, about 84 to about 88, about 84 to about 86, about 87 to about 100, or about 87 to about 90° C).

Preferably the lactose-containing liquid stream 4 is heated to about 75° C to about 85° C, and more preferably to about 75° C.

In one embodiment the lactose-containing liquid stream is held at temperature for about for about 30 seconds, 1, 2, 3, 4, 5, 6, 7, 8, 9, 10, 11, 12, 13, 14, 15, 16, 17, 18, 19 or 20 minutes. In one embodiment the lactose-containing liquid stream is held at temperature for about 2 to about 5 minutes and more preferably about 3 minutes

In one embodiment, once heated to the desired temperature the temperature of the lactose-containing liquid stream is allowed to drop to a lower temperature. Generally this occurs once all of the lactose is dissolved in the lactose-containing liquid stream. For example, in one embodiment the temperature may be allowed to decrease to a lower temperature of about 65° C to about 90° C, 65° C to about 85° C, or 70° C to about 85° C, provided the temperature is sufficient to ensure that substantially all of the lactose remains in solution.

In one embodiment, once heated, the lactose-containing liquid stream is held at the lower temperature for about 30 seconds, 1, 2, 3, 4, 5, 6, 7, 8, 9, 10, 11, 12, 13, 14, 15, 16, 17, 18, 19 or 20 minutes. In one embodiment the lactose-containing liquid stream is held at temperature for about 2 to about 5 minutes and more preferably about 3 minutes.

In one embodiment, once the lactose-containing liquid stream 4 is heated the pH is adjusted.

In an alternate embodiment the lactose-containing liquid stream 4 is pH adjusted before heating.

In one embodiment the pH is adjusted to about 6.7, 6.8, 6.9, 7.0, 7.1, 7.2, 7.3, 7.4, 7.5, 7.6, 7.7, 7.8, 7.9 or 8.0, and useful ranges may be selected between any of these values (for example, from about 6.7 to about 8.0, about 6.7 to about 7.8, about 6.7 to about 7.6, about 6.7 to about 7.5, about 6.7 to about 7.1, about 6.7 to about 6.9, about 6.9 to about 8.0, about 6.9 to about 7.8, about 6.9 to about 7.5, about 6.9 to about 7.2, about 7.0 to about 8.0, about 8.0 to about 7.9, about 7.0 to about 7.7, about 7.0 to about 7.5, about 7.0 to about 7.4, about 7.0 to about 7.3, about 7.0 to about 7.2, about 7.1 to about 8.0, about 7.1 to about 7.8, about 7.1 to about 7.5, about 7.1 to about 7.4, about 7.1 to about 7.3, about 7.1 to about 7.2, about 7.2 to about 8.0, about 7.2 to about 7.8, about 7.2 to about 7.6, about 7.2 to about 7.4, about 7.5 to about 8.0, about 7.5 to about 7.8, about 7.5 to about 7.7, about 7.8 to about 8.0).

Preferably the pH is adjusted to about 7.0 to about 7.2.

Various methods may be used to adjust the pH. For example, the pH may be adjusted by adding an acid or base, such as a food grade acid or food grade base.

Generally, the lactose-containing liquid stream 4 is acidic. For example, the lactose-containing liquid stream may have a pH of between about 5.6 and 6, and more typically around 5.8. In this instance the pH is adjusted by adding a base. For example, the base may be a food grade base. The base may be selected from KOH or NaOH. The pH adjuster may be in a concentrated or dilute form. For example, the base may be selected from concentrated KOH or NaOH, or dilute KOH or NaOH.

The preferred and tested means of pH adjustment is by injection of KOH or NaOH in-line with a static mixer immediately downstream of the injection point. A less preferred alternative is to add the KOH or NaOH to the heated lactose-containing liquid stream in an agitated vessel.

In one embodiment the lactose-containing liquid stream is agitated, mixed or pumped with turbulent flow during heating and/or pH adjustment.

### 3.2 Centrifugal separation

In one embodiment the lactose-containing liquid stream 4, optionally heated and/or pH adjusted 5, is passed through a centrifugal separator 6 to separate the light and heavy phases. The centrifugal separator 6 can be selected from a clarifier or a decanter. Preferably the separator is a clarifier.

In various embodiments, the light phase is substantially free of precipitated calcium species, including, but not limited to calcium phosphate and calcium sulphate, or the lactose content of the light phase is greater than the lactose content of the heavy phase, and combinations thereof.

In one embodiment subjecting the pH adjusted and/or heated lactose-containing liquid stream to centrifugal separation removes at least 60, 65, 70, 75, 80, 85, 90, 95% by weight of the calcium from the lactose-containing liquid stream, and useful ranges may be selected between any of these values (for example, about at least 60 to about 95, about 60 to about 85, about 60 to about 80, about 60 to about 70, about 65 to about 95, about 65 to about 90, about 65 to about 85, about 65 to about 80, about 65 to about 70, about 70 to about 95, about 70 to about 90, about 70 to about 85, about 70 to about 80, about 75 to about 95, about 75 to about 90, about 75 to about 85, about 80 to about 95, about 80 to about 90 or about 90 to about 95% by weight calcium removal from the lactose-containing liquid stream).

In one embodiment the centrifugal separator 6 is capable of generating at least 10,000 G, and preferably about 12,000 to about 16,000 G.

The separator feed stream should be at a sufficient temperature to ensure that substantially all of the lactose remains in solution. In some embodiments the separator feed stream temperature is about 65° C to a bout 100° C, and more preferably about 65° C to about 90° C, and more preferably about 70° C to about 85° C.

In one embodiment the pH of the separator feed stream is about 6.7 to about 8.0, and more preferably about 7.0 to about 7.2. In some embodiments a pH adjustor is added to adjust the pH of the separator feed stream to the desired pH. For example, if the pH needs to be adjusted NaOH or KOH may be added. In some embodiments the pH adjustor is added at the inlet to the separator.

The concentration of suspended solids (precipitate) in the separator feed stream should be such that it does not cause overloading of the separator.

In some embodiments a useful indication of an optimal dilution ratio is the calcium concentration in the diluted separator feed stream. For example, in some embodiments, a calcium concentration of about 0.2% by weight to about 0.6% by weight, and more preferably 0.4% by weight, is an indicator of an optimal dilution ratio.

In one embodiment the concentration of the suspended solids in the separator feed stream is diluted. In one embodiment the concentration of the suspended solids in the separator feed stream is diluted by recycling a fraction of the light phase from the separator into the incoming separator feed stream. For example, the recycled fraction may comprise about 40% to about 70%, and more preferably about 55%, of the liquid stream volumetric flow.

In an alternate embodiment the separator feed stream is diluted by adding water, milk permeate, whey permeate or any combination of any two or more thereof. In some embodiments an amount of water is added to achieve a calcium concentration in the separator feed stream of about 0.2% by weight to about 0.6% by weight, and more preferably 0.4% by weight. If diluting the separator feed stream with water, the temperature and pH are maintained within the acceptable ranges as described above.

In one embodiment the heavy phase from the separator is sent to waste or is further processed.

In some embodiments the heavy phase is further processed by dilution with water (as explained above) and processing through a centrifugal separator. Carrying out this process enables greater lactose recovery, but may also result in greater processing volumes and more dilute processing volumes at downstream processing steps.

### 3.3 Filtration

The processed lactose-containing liquid stream is subjected to filtration 7 to remove at least some non-lactose components from the lactose-containing liquid stream.

In those embodiments where the lactose-containing liquid stream is first processed by centrifugal separation 6, the processed lactose-containing liquid stream is then processed by ultrafiltration (UF) and/or nanofiltration (NF).

When the lactose-containing liquid stream is processed by ultrafiltration the retentate is removed and the UF permeate is obtained for use.

When the lactose-containing liquid stream is processed by nanofiltration the permeate is removed and the NF retentate is obtained for use.

In one embodiment the lactose-containing liquid stream is processed by multiple nanofiltration steps. In such embodiments the retentate of the first nanofiltration step is removed and the NF permeate used in the subsequent nanofiltration step or steps. With the final nanofiltration step the permeate is removed and the NF retentate is obtained for use.

In one embodiment the processed lactose-containing liquid stream is first processed by ultrafiltration, in which the retentate is removed, and the UF permeate is obtained for filtration by NF. Following NF of the UF permeate, the NF permeate is removed and the NF retentate is obtained for use.

### 3.3.1 Ultrafiltration (UF)

Ultrafiltration can be used after centrifugal separation, or in place of centrifugal separation.

Ultrafiltration is carried out to remove particles from the UF permeate that are larger than lactose. For example, cells, proteins, polypeptides, polysaccharides, lipid, or precipitated salts.

In some embodiments the molecular weight cut-off (MWCO) of the UF membrane is about 1,000 Daltons to about 10,000 Daltons, and preferably about 3,000 Daltons to about 5,000 Daltons.

In one embodiment the UF membrane is selected from a spiral membrane, ceramic membrane, hollow fibre membrane, or a flat sheet membrane.

Typically the non-ceramic membranes are polymer membranes formed from, for example, polysulfone, polypropylene, or cellulose acetate.

In some embodiments the UF is carried out with a ceramic membrane. Ceramic membranes can be subjected to a higher heat than polymer membranes.

Before ultrafiltration, in some embodiments the lactose-containing liquid stream is pH adjusted and/or heated. In some embodiments ultrafiltration removes at least 60, 65, 70, 75, 80, 85, 90, 95% by weight of the calcium from the lactose-containing liquid stream, and useful ranges may be selected between any of these values (for example, about at least 60 to about 95, about 60 to about 85, about 60 to about 80, about 60 to about 70, about 65 to about 95, about 65 to about 90, about 65 to about 85, about 65 to about 80, about 65 to about 70, about 70 to about 95, about 70 to about 90, about 70 to about 85, about 70 to about 80, about 75 to about 95, about 75 to about 90, about 75 to about 85, about 80 to about 95, about 80 to about 90 or about 90 to about 95% by weight calcium removal from the lactose-containing liquid stream).

In some embodiments the lactose-containing liquid stream is diluted with water, preferably demineralised water. For example, the lactose-containing liquid stream can be diluted at a ratio of between about 1:2 to about 1:0.5 mother liquor to water. Preferably the dilution is at a ratio of about 1:1 mother liquor to water.

If spiral membranes are used, the lactose-containing liquid stream prior to UF should preferably be at a temperature of about 10° C to about 50° C, more preferably about 45° C to about 50° C, and most preferably about 45° C.

In some embodiments, a polymer membrane is used for ultrafiltration and the permeate flux during UF is 4, 5, 6, 7, 8, 9, 10, 11, 12, 13, 14, 15, 16, 17, 18, 19 or 20 litres per hour per m² (LMH), and useful ranges may be selected between any of these values (for example, about 4 to about 20, about 4 to about 16, about 4 to about 12, about 4 to about 8, about 6 to about 20, about 6 to about 18, about 6 to about 17, about 6 to about 13, about 6 to about 11, about 7 to about 20, about 7 to about 19, about 7 to about 15, about 7 to about 13, about 7 to about 10, about 8 to about 20, about 8 to about 14, about 8 to about 10, about 9 to about 20, about 9 to about 18, about 9 to about 16, about 9 to about 12, about 11 to about 20, about 11 to about 18, about 11 to about 14, about 12 to about 20, about 12 to about 19, about 12 to about 17, about 12 to about 15, about 13 to about 20, about 13 to about 70, about 13 to about 15, about 15 to about 2,0 15 to about 18, about 15 to about 16 or about 16 to about 20 LMH).

If cooling of the lactose-containing liquid stream prior to UF is required, then any heat exchanger known in the art could be used, such as a plate heat exchanger.

In some alternate embodiments a high-temperature tolerant UF membrane is used, such as a high-temperature tolerant ceramic UF membrane. In this embodiment the temperature of the lactose-containing liquid stream may be up to about 95° C, and more preferably about 75° C.

In some embodiments, a high-temperature tolerant UF membrane is used for ultrafiltration and the permeate flux UF is at least 40, 45, 50, 55, 60, 65, 70, 75, 80, 85, 90, 95, 100, 105, 110, 115, 120 litres per hour per m² (LMH), and useful ranges may be selected between any of these values (for example, about 40 to about 120, about 40 to about 95, about 40 to about 65, about 45 to about 120, about 45 to about 115, about 45 to about 110, about 45 to about 95,0, about 45 to about 70, about 55 to about 120, about 55 to about 110, about 55 to about 100, about 55 to about 75, about 60 to about 120, about 60 to about 90, about 60 to about 80, about 65 to about 120, about 65 to about 110, about 65 to about 100, about 65 to about 85, about 70 to about 120, about 70 to about 110, about 70 to about 100, about 70 to about 80, about 75 to about 120, about 75 to about 115, about 75 to about 105, about 75 to about 95, about 80 to about 120, about 80 to about 110, about 80 to about 100, about 85 to about 120, about 85 to about 110, about 85 to about 100, about 95 to about 120, about 95 to about 115, about 95 to about 100 or about 100 to about 120 LMH).

In some embodiments UF concentrates the retentate stream to about 24% to about 29% total solids, and more preferably about 25% total solids.

In some embodiments diafiltration (DF) with water is used to increase the recovery of lactose in the UF permeate stream. In some embodiments diafiltration is used with UF to obtain a volume concentration factor (VCF) of about 20 to about 30, and more preferably about 25, to obtain a lactose yield (in the permeate) for the UF step of about 95%.

The UF permeate stream not be allowed to cool below about 40° C as this may risk lactose crystal formation.

In some embodiments the UF retentate is sent to waste.

### 3.3.2 Nanofiltration (NF)

The molecular weight cut-off of an NF membrane is typically less than 1000 Daltons.

In one embodiment the NF membrane is selected from a spiral membrane, ceramic membrane, or hollow fibre membrane. In one embodiment the NF membrane is a spiral membrane. In an alternate embodiment the NF membrane is a ceramic membrane.

In one embodiment the UF permeate is processed by NF.

In one embodiment the nanofiltration comprises two or more nanofiltration steps. The first nanofiltration step may be carried out with a molecular weight cutoff higher than the second and subsequent nanofiltration steps. For example, in the case of a two stage nanofiltration process the first nanofiltration may be carried out with a molecular weight cutoff of at about 1000 Daltons, and the second nanofiltration may be carried out with a molecular weight cutoff of less than 250 Daltons.

In one embodiment the tested nominal rejection of the NF membranes is about 96% to about 99% MgSO₄ rejection.

The temperature of the NF feed stream should be about 10° C to about 50° C, preferably about 45° C to about 50° C, and more preferably about 45° C.

If cooling of the NF feed stream is required, then any heat exchanger known in the art could be used, such as a plate heat exchanger.

In one embodiment the NF increases the concentration of the retentate stream up to about 35% total solids. Preferably the NF increases the concentration of the retentate stream to about 25% to about 29% total solids, and more preferably about 26% to about 27% total solids.

In one embodiment diafiltration with water is used to increase the removal of ash into the NF permeate stream. In some embodiments diafiltration is used with NF to obtain a volume concentration factor (VCF) of about 8 to about 12, and more preferably about 10, to obtain a lactose yield (in the retentate) for the NF step of about 90%.

In an alternate embodiment diafiltration is carried out with HCI. In one embodiment dilute HCI is used. Preferably the concentration of HCl is about 0.0575% by weight to 0.0675% by weight.

The shift in pH alters the ion balance so that more of the mineral content is released in the permeate. The use of HCl specifically allows maintenance of the NF flux at higher solids owing to the presence of added chloride ions.

In one embodiment the pH of the NF feed stream is adjusted before NF. In some embodiments the pH is adjusted to about 5.8 to about 6, and more preferably about 5.85 to about 5.95.

In one embodiment concentrated HCl is used to adjust the pH of the NF feed stream. In some embodiments about 0.4% to about 1% of concentrated HCI by volume is added to the feed stream. The concentrated HCl typically has a concentration of about 25% w/v to about 40% w/v, and preferably between about 30% w/v by weight and 35% w/v. In some embodiments the concentrated HCI has a concentration of about 33% w/v.

In one embodiment diluted HCI is used to adjust the pH of the NF feed stream. For example, about 10% by weight to about 20% by weight dilute HCI can be added to the NF feed stream.

In one embodiment the HCl is injected in-line. For example, the HCl may be injected in-line. In some embodiments a static mixer is present immediately downstream of the injection point. In an alternate embodiment the HCI is added in an agitated vessel.

In some embodiments the NF permeate is sent to waste.

### 4. Lactose-containing extract

In one embodiment the lactose-containing extract comprises at least about 73, 74, 75, 76, 77, 78, 79, 80, 81, 82, 83, 84 or 85% lactose on a dry basis, and useful ranges may be selected between any of these values (for example, about 73 to about 85, about 73 to about 84, about 73 to about 81, about 73 to about 80, about 73 to about 77, about 73 to about 75, about 74 to about 85, about 74 to about 80, about 74 to about 76, about 75 to about 85, about 75 to about 80, about 75 to about 77, about 78 to about 85, about 78 to about 82, about 78 to about 80, about 78 to about 79, about 80 to about 85, about 80 to about 83, about 80 to about 81, about 81 to about 85, about 81 to about 84, about 83 to about 85% by weight lactose on a dry basis).

In one embodiment the lactose-containing extract comprises at least about 20% by weight to about 30% by weight total solids.

In one embodiment the UF permeate stream feeds into NF. In such embodiments the pH of the UF permeate stream may be adjusted as described above.

In some embodiments the lactose-containing extract has a SNL/TS ratio of less than 0.15, 0.16, 0.17, 0.18, 0.19, 0.20, 0.21, 0.22, 0.23, 0.24, 0.25, 0.26, 0.27, 0.28, 0.29 or 0.30, and useful ranges may be selected between any of these values (for example, a ratio of about 0.15 to about 0.30, about 0.15 to about 0.28, about 0.15 to about 0.25, about 0.15 to about 0.20, about 0.16 to about 0.30, about 0.16 to about 0.28, about 0.16 to about 0.26, about 0.16 to about 0.24, about 0.16 to about 0.20, about 0.17 to about 0.20, about 0.17 to about 0.29, about 0.17 to about 0.28, about 0.17 to about 0.27, about 0.17 to about 0.25, about 0.17 to about 0.21, about 0.17 to about 0.20, about 0.18 to about 0.30, about 0.18 to about 0.29, about 0.18 to about 0.27, about 0.18 to about 0.26, about 0.18 to about 0.25, about 0.18 to about 0.22, about 0.18 to about 0.20, about 0.19 to about 0.30, about 0.19 to about 0.28, about 0.19 to about 0.27, about 0.19 to about 0.25, about 0.19 to about 0.23, about 0.20 to about 0.30, about 0.20 to about 0.28, about 0.20 to about 0.27, about 0.20 to about 0.26, about 0.2 to about 0.24, about 0.21 to about 0.30, about 0.21 to about 0.28, about 0.21 to about 0.26, about 0.21 to about 0.25, about 0.23 to about 0.30, about 0.23 to about 0.27, about 0.25 to about 0.30, about 0.25 to about 0.28, about 0.27 to about 0.30 or about 0.28 to about 0.30 SNL/TS).

In some embodiments the SNL/TS ratio of the lactose-containing extract is substantially the same as the lactose-containing liquid source.

In one embodiment at least a portion of the lactose-containing extract is added to the lactose-containing liquid source.

In one embodiment a majority of the lactose-containing extract is added to the lactose-containing liquid.

In one embodiment substantially all of the lactose-containing extract is added to the lactose-containing liquid.

In one embodiment at least 60, 65, 70, 75, 80, 85, 90, 95 or 99% of the lactose-containing extract is added to the lactose-containing liquid, and useful ranges may be selected between any of these values (for example, from about 60 to about 99, from 60 to about 95, from 90 to about 90, from 60 to about 80, from 65 to about 99, from 65 to about 80, from 65 to about 70, from 75 to about 99, from 75 to about 95, from 75 to about 85, from 80 to about 99, from 80 to about 95, from 80 to about 85, from 85 to about 99, from 85 to about 95, from 85 to about 90, from 90 to about 99, from 90 to about 95%).

In one embodiment the lactose-containing extract is added to the lactose-containing liquid source at a rate of about 10, 12, 14, 16, 18, 20, 22, 24, 26, 28, 30% of lactose source volumetric flow, and useful ranges may be selected between any of these values (for example, about 10 to about 30, about 10 to about 26, about 10 to about 18, about 10 to about 16, about 12 to about 30, about 12 to about 26, about 12 to about 24, about 12 to about 20, about 12 to about 18, about 14 to about 30, about 14 to about 20, about 16 to about 30, about 16 to about 28, about 13 to about 24, about 16 to about 20, about 20 to about 30, about 20 to about 28, about 20 to about 26, about 20 to about 24, about 22 to about 30, about 22 to about 24, about 24 to about 30, about 24 to about 26, about 26 to about 30 or about 28 to about 30% of lactose-containing liquid source volumetric flow).

In some alternate embodiments the lactose-containing extract is used in other processes. For example, the lactose-containing extract may be used to standardise milk products (such as milk powder), or may be used in an ethanol production process.

The use of the method of the present invention reduces the amount of lactose lost as waste. This increases the lactose yield per unit of lactose-containing liquid source. We have found that the present method increases lactose yield from the lactose-containing liquid source to at least about 70, 72, 74, 76, 78, 80, 82, 84, 86, 88, 90, 92, 94 or 95% to about 95% and useful ranges may be selected between any of these values (for example, from about 70 to about 95, about 70 to about 91, about 70 to about 87, about 70 to about 85, about 70 to about 82, about 70 to about 79, about 70 to about 75, about 70 to about 71, about 73 to about 95, about 73 to about 90, about 73 to about 88, about 73 to about 86, about 73 to about 82, about 73 to about 80, about 73 to about 79, about 73 to about 77, about 73 to about 75, about 75 to about 95, about 75 to about 90, about 75 to about 88, about 75 to about 85, about 75 to about 81, about 75 to about 78, about 79 to about 95, about 79 to about 92, about 79 to about 88, about 79 to about 86, about 79 to about 84, about 79 to about 81, about 82 to about 95, about 82 to about 91, about 82 to about 90, about 82 to about 89, about 82 to about 86, about 85 to about 95, about 85 to about 92, about 85 to about 90, about 85 to about 88, about 89 to about 95, about 89 to about 92, about 89 to about 90, about 91 to about 95%).

In one embodiment the lactose concentration, by weight of the total solids, in the lactose-containing extract is 8, 10, 12, 14, 16, 18, 20, 22, 24, 26, 28, 30, 32, 34, 36, 38, 40, 42, 44 or 46% higher than in the lactose-containing liquid stream, and useful ranges may be selected between any of these values (for example, from about 8 to about 46, about 8 to about 44, about 8 to about 38, about 8 to about 30, about 8 to about 28, about 8 to about 26, about 8 to about 24, about 8 to about 20, about 8 to about 18, about 12 to about 46, about 12 to about 44, about 12 to about 40, about 12 to about 30, about 12 to about 28, about 12 to about 26, about 12 to about 20, about 12 to about 18, about 18 to about 46, about 18 to about 44, about 18 to about 42, about 18 to about 36, about 18 to about 32, about 18 to about 28, about 18 to about 26, about 18 to about 22, about 18 to about 20, about 20 to about 46, about 20 to about 44, about 20 to about 38, about 20 to about 34, about 20 to about 28, about 20 to about 26, about 24 to about 46, about 24 to about 44, about 24 to about 32, about 24 to about 28, about 26 to about 46, about 26 to about 40, about 26 to about 32, about 26 to about 30, about 26 to about 28, about 32 to about 46, about 32 to about 40, about 36 to about 46, about 36 to about 42, about 40 to about 46, about 40 to about 42 or about 42 to about 46% by weight of total solids).

In one embodiment the lactose concentration, by weight of the total solids, in the lactose-containing extract is at least 70, 75, 80, 85, 90, 95, 100, 105, 110% of the lactose-containing liquid source, and useful ranges may be selected between any of these values (for example, from about 70 to about 110, about 70 to about 100, about 70 to about 90, about 70 to about 80, about 75 to about 110, about 75 to about 105, about 75 to about 95, about 75 to about 80, about 80 to about 110, about 80 to about 100, about 80 to about 95, about 80 to about 90, about 85 to about 110, about 85 to about 100, about 85 to about 95, about 90 to about 110, about 90 to about 105, about 90 to about 100, about 95 to about 110, about 95 to about 100, about 100 to about 110% by weight of the lactose concentration in the lactose-containing liquid source).

### 5. Examples

### Example 1 - Standard lactose processing

A lactose-containing liquid source was obtained as a by-product of cheese manufacture or casein manufacture. The lactose-containing liquid source was ultrafiltered and the whey permeate (containing lactose and minerals) was concentrated by reverse osmosis to a total solids of 18% by weight to about 20% by weight. The concentration of lactose was approximately 80% of the total solids.

The lactose-containing liquid was further concentrated to 60% total solids in an evaporator.

The lactose-containing liquid was then transferred to a cooling crystalliser. Following crystallisation the crystal solids were separated from the smaller crystals and liquid phase by centrifugation. The crystal solids were then washed and then further processed to a final lactose product. The mother liquor (containing the smaller lactose crystals and liquid phase) is then typically used in animal feed.

The inventors have found that such processes lead to a loss of approximately 30% of the lactose in the process feed stream to the mother liquor stream.

### Example 2 - Nanofiltration

As in example 1, a lactose-containing liquid source is subjected to lactose crystallisation.

In contrast to example 1, the lactose-containing liquid stream is also subjected to nanofiltration with a pore size of 1 nm. The permeate is collected and then subjected to a further nanofiltration step with a pore size of 0.25 nm. The retentate is collected to obtain the lactose-containing extract.

The lactose-containing extract has a higher lactose concentration by weight of total solids, compared to the lactose-containing liquid stream of Example 1.

### Example 3 - Ultrafiltration

As in example 1, a lactose-containing liquid source was subjected to lactose crystallisation.

The mother liquor was first heated to about 75° C by direct steam injection to dissolve substantially all of the lactose in the mother liquor and was held at this heat for about 3 min.

The heated mother liquor was then pH adjusted by the addition of concentrated NaOH to achieve a pH of about 7.0.

The pH adjusted and heated mother liquor was then subjected to ultrafiltration using a ceramic membrane with a nominal pore size of 5 nm and a feed temperature of about 75° C to about 80° C. The UF concentrated the retentate stream to about 27% total solids which was then disposed of as waste. The combination of heating, pH adjustment and UF removed greater than 96% of the calcium from the mother liquor.

### Example 4 - Ultrafiltration and nanofiltration

As in example 1, a lactose-containing liquid source was subjected to lactose crystallisation. The plant used to conduct this example comprised a balance tank, recirculation pump, membrane filter and associated instrumentation and controls. The balance tank was filled with mother liquor that had been pre-treated with heat treatment and pH adjustment to precipitate dissolved salts. The pH was adjusted to 7.2 with NaOH and was subjected to a heat and hold process (∼80°C for 10 min) before dilution (∼1:1 dilution with hot demineralised water) and processing by UF using either a spiral polymer membrane or ceramic membrane.

The pre-treated mother liquor was pumped to the membrane filter through which some of the water, lactose and dissolved salts permeated, with the balance of the liquor containing the precipitated salts and high molecular weight material being returned to the balance tank. In this way the flux, or volumetric rate of liquor passing through the membrane per unit area of filter, was measured as the concentration of precipitated salts and high molecular weight material increased. Diafiltration water was added at different stages of concentration to improve lactose recovery though the membrane.

Ultrafiltration was carried out using either a polymer membrane or a ceramic membrane. The polymer membrane had a nominal pore size of 5 kDa, a feed temperature of about 49°C, and an inlet pressure of about 2.4 bar. Additionally the feed was processed by centrifugation.

The ceramic membrane had a nominal pore size of 5 nm, a feed temperature of about 80° C, maximal cross flow and 2.0 bar inlet pressure.

The UF concentrated the retentate stream to about 28% total solids which was then disposed of as waste.

**Table 1. Permeate flux rates achieved during ultrafiltration with a polymer membrane**

| **Elapsed time** | **Permeate flux** |
|---|---|
| **(min)** | **(LMH)** |
| 15 | 16.0 |
| 30 | 13.6 |
| 60 | 12.3 |
| 90 | |
| 90 | 11.3 |
| 120 | 10.5 |
| 150 | 9.4 |
| 180 | 7.7 |
| 180 | |
| 210 | 7.7 |
| 240 | 7.9 |
| 270 | 7.8 |
| 300 | 7.8 |

**Table 2. Analysis of the content of the UF samples when using a polymer membrane during ultrafiltration.**

| **UF sample** | **Total solids** | **Lactose** | **Ash** | **Total Nitrogen** | **Non-protein nitrogen** | **True protein** | **Calcium** |
|---|---|---|---|---|---|---|---|
| | **%** w/w | **% w/w** | **% w/w** | **% w/w** | **% w/w** | **% w/w** | **mg/kg** |
| Mother liquor | 34.1 | 19.6 | 9.6 | 0.62 | 0.54 | 0.50 | 7200 |
| UF feed | 12.5 | 7.7 | 3.5 | 0.23 | 0.20 | 0.15 | 139 |
| UF retentate - VCF | 17.3 | 7.8 | 3.7 | 0.87 | 0.32 | 3.50 | 173 |
| UF permeate - VCF | 13.4 | 8.1 | 3.9 | 0.23 | 0.23 | 0.02 | 163 |
| UF bulk permeate | 12.2 | 7.3 | 3.6 | 0.19 | 0.20 | -0.05 | 135 |

**Table 3. Permeate flux rates achieved during ultrafiltration with a ceramic membrane**

| **Elapsed Time** | **Crossflow** | **Permeate flux** |
|---|---|---|
| **(min)** | **(m/s)** | **(LMH)** |
| 0 | 6.2 | |
| 15 | 6.2 | 50.4 |
| 30 | 6.2 | 54.0 |
| 57 | 6.2 | 54.0 |
| 90 | 6.1 | 67.2 |
| 120 | 5.9 | 82.8 |
| | | |
| 150 | 5.9 | 90.0 |
| | | |
| 180 | 5.8 | 96.0 |
| | | |
| 210 | 5.8 | 96.0 |
| | | |
| 230 | 5.8 | 90.0 |
| 235 | 5.5 | 115.2 |
| 245 | 5.5 | 115.2 |
| 300 | 5.5 | 117.6 |

**Table 4. Analysis of the content of the UF samples when using a ceramic membrane during ultrafiltration.**

| **UF sample** | **Total solids % w/w** | **Lactose % w/w** | **Ash % w/w** | **Total Nitrogen % w/w** | **Total protein % w/w** | **Non-protein nitrogen % w/w** | **Calcium mg/kg** |
|---|---|---|---|---|---|---|---|
| Mother liquor | 37.5 | 21.8 | 10.80 | 0.710 | 0.734 | 0.595 | 7330 |
| UF feed | 21.1 | 12.0 | 6.83 | 0.379 | 0.140 | 0.357 | 3910 |
| Retentate - 57 min | 22.2 | 12.1 | 7.34 | 0.415 | 0.396 | 0.353 | 5250 |
| Permeate - 57 min | 19.9 | 12.5 | 5.89 | 0.315 | -0.045 | 0.322 | 143 |
| Retentate - 125 min | 15.0 | 8.1 | 5.22 | 0.299 | 0.453 | 0.228 | 5560 |
| Permeate - 125 min | 12.4 | 7.6 | 3.61 | 0.199 | 0.019 | 0.196 | 64.4 |
| Retentate - 158 min | 17.0 | 7.9 | 6.40 | 0.367 | 0.778 | 0.245 | 8770 |
| Permeate - 158 min | 12.8 | 7.6 | 3.72 | 0.210 | 0.064 | 0.200 | 58.4 |
| Retentate - 191 min | 12.0 | 4.5 | 5.08 | 0.290 | 0.791 | 0.166 | 9080 |
| Permeate - 191 min | 7.3 | 4.5 | 2.09 | 0.117 | -0.026 | 0.121 | 37.5 |
| Retentate - 210 min | 14.9 | 4.5 | 7.00 | 0.399 | 1.410 | 0.178 | 14300 |
| Permeate - 210 min | 7.7 | 4.8 | 2.15 | 0.130 | -0.019 | 0.133 | 36.6 |
| Retentate - 230 min | 11.9 | 2.6 | 6.28 | 0.335 | 1.321 | 0.128 | 14900 |
| Permeate - 230 min | 4.6 | 2.8 | 1.27 | 0.07 | -0.064 | 0.082 | 25.9 |
| UF bulk permeate | 13.0 | 7.8 | 3.85 | 0.209 | -0.108 | 0.226 | 83.9 |
| Yield in bulk permeate | | 88.0 | 76.4 | | 0.0 | | 2.9 |

The UF permeate was collected and subjected to nanofiltration using a standard sanitary spiral membrane with a feed temperature of about 45° C. The tested nominal rejection of the NF membranes is about 96 to about 99% MgSO₄ rejection. If necessary the NF feed stream is pH adjusted to about 5.85 to about 5.95 using concentrated HCI.

The NF permeate was sent to waste.

The NF retentate comprised about 70% by weight to 80% by weight lactose on a dry basis.

As shown in Table 1, flux rates of 8 litres per hour per m² (LMH) of membrane area were obtained for the polymer membranes. As shown in Table 3 the flux rates of 70 LMH were achieved using the ceramic membranes.

This process results in less lactose being lost in the mother liquor. For example, running this process leads to a loss of approximately 5% of the lactose in the process feed stream to the waste streams, which is an improvement over the standard process described in Example 1.

Additionally, as shown in Tables 2 and 4, lactose can be recovered from the lactose-containing liquid source and returned to the crystalliser on a continuous basis while controlling the amount of the solids non-lactose.

### Example 5 - Centrifugal separation and nanofiltration

As in example 1, a lactose-containing liquid source is subjected to lactose crystallisation.

The mother liquor is first heated to about 75° C by direct steam injection to dissolve substantially all of the lactose in the mother liquor. Once the lactose is dissolved the temperature is allowed to drop but is kept at a temperature to retain the lactose in solution.

The heated mother liquor is then pH adjusted by the addition of concentrated KOH to achieve a pH between about 7 and 7.2.

The pH adjusted mother liquor is then held at about 70° C for about 3 minutes.

The heated, pH adjusted mother liquor is then passed through a clarifier running at 14,000 G to split the light and heavy phases. The separator feed stream is at a temperature of between 65° C to 85° C. The combination of heating, pH adjustment and centrifugal separation removes greater than 95% of the calcium from the mother liquor.

The heavy phase from the separator is sent to waste or is further processed.

The light phase is then subjected to nanofiltration using a standard sanitary spiral membrane with a feed temperature of about 45° C. The tested nominal rejection of the NF membranes is about 96 to about 99% MgSO₄ rejection. If necessary the NF feed stream is pH adjusted to about 5.85 to about 5.95 using concentrated HCI.

The NF permeate is collected and subjected to nanofiltration using a standard sanitary spiral membrane with a feed temperature of about 45° C. The tested nominal rejection of the NF membranes is about 96 to about 99% MgSO₄ rejection. If necessary the NF feed stream is pH adjusted to about 5.85 to about 5.95 using concentrated HCI.

The NF permeate is sent to waste.

The NF retentate comprises about 65% by weight to 70% by weight lactose on a dry basis.

This lactose in the NF retentate can be used in some processes requiring a concentrated lactose stream, for example as a feed stream in ethanol production.

Alternately, the lactose in the NF retentate is blended with the lactose-containing liquid source at a rate of about 10% to about 20% of lactose-containing liquid source volumetric flow.

This process results in less lactose being lost in the mother liquor. For example, running our process leads to a loss of approximately 5% of the lactose in the process feed stream to the waste streams, which is an improvement over the standard process as described in example 1.

### Example 6 - Processing of mother liquor

As in example 1, a lactose-containing liquid source was subjected to lactose crystallisation.

In contrast to example 1, the mother liquor was collected for processing.

The mother liquor was first heated to about 75° C by direct steam injection to dissolve substantially all of the lactose in the mother liquor.

The heated mother liquor was then pH adjusted by the addition of concentrated KOH to achieve a pH between about 7 and 7.2.

The pH adjusted mother liquor was then held at about 70° C for about 3 minutes.

The heated, pH adjusted mother liquor was then passed through a clarifier running at 14,000 G to split the light and heavy phases. The separator feed stream was at a temperature of between 65° C to 85° C.

The heavy phase from the separator can be sent to waste or can be further processed.

The light phase was then subjected to ultrafiltration using a standard sanitary spiral membrane with a molecular weight cut-off (MWCO) of 5,000 Daltons and a feed temperature of about 45° C. The combination of heating, pH adjustment and UF removed greater than 96% of the calcium from the mother liquor. The UF concentrated the retentate stream to about 25% total solids which was then disposed of as waste.

This demonstrated that the heated, pH adjusted, spiral membrane UF extract (the UF permeate) had a very similar composition to mother liquor subjected to heating, pH adjustment and UF with a ceramic membrane.

The UF permeate was collected and subjected to nanofiltration using a standard sanitary spiral membrane with a feed temperature of about 45° C. The tested nominal rejection of the NF membranes was about 96 to about 99% MgSO₄ rejection. If necessary the NF feed stream was pH adjusted to about 5.85 to about 5.95 using concentrated HCI.

The NF permeate was sent to waste.

The NF retentate comprised about 73% by weight to 85% by weight lactose on a dry basis.

This lactose in the NF retentate can be used in some processes requiring a concentrated lactose stream, for example as a feed stream in ethanol production.

Alternately, the lactose in the NF retentate can be blended with the lactose-containing liquid source at a rate of about 10% to about 20% of lactose-containing liquid source volumetric flow.

This process results in less lactose being lost in the mother liquor. For example, running our process leads to a loss of approximately 7% of the lactose in the process feed stream to the waste streams, which is an improvement over the standard process described in example 1.

### Example 7 - Processing of mother liquor and wash water

Lactose-containing liquid source was crystallised as per example 6. However, in this example wash water, as well as mother liquor was collected and subjected to centrifugal separation, ultrafiltration and nanofiltration.

An advantage of this process is that the wash water was "cleaned" before reprocessing to remove non-lactose material. An advantage of this "cleaning" is that it reduces the processing load in the successive processing steps.

## Claims

1. A method of obtaining lactose from a lactose-containing liquid source comprising
subjecting a lactose-containing liquid source to a lactose crystallisation process to generate one or more lactose-containing liquid streams,
subjecting the one or more lactose-containing liquid streams to (i) heat, (ii) pH adjustment, or (iii) heat and pH adjustment,
subjecting the one or more lactose-containing liquid streams to one or more processes to generate a lactose-containing extract, the one or more processes comprising
i) a first step, to remove one or more of cells, proteins, polypeptides, polysaccharides, lipid, ions or salts, comprising
a) centrifugal separation to produce a light phase and a heavy phase with optional ultrafiltration or nanofiltration of the light phase, or
b) ultrafiltration to generate an ultrafiltration permeate and an ultrafiltration retentate, and
ii) a second step comprising nanofiltration of
a) the light phase from the centrifugal separator, or
b) the permeate from ultrafiltration, or
c) the retentate from nanofiltration,
adding at least a portion of the lactose-containing extract to the lactose-containing liquid source before, during or after entry into the crystallisation process.

2. A method of claim 1 wherein the lactose-containing liquid stream is subjected to
i) heating to a first temperature, or
ii) pH adjustment, or
iii) heating to a first temperature and pH adjustment, or
iv) pH adjustment and heating to a first temperature,
before processing by centrifugation or filtration.

3. A method of claim 2 wherein the lactose-containing liquid stream is heated to a first temperature of 70° C to 100° C.

4. A method of claim 3 wherein the lactose-containing liquid stream is held at the heated temperature for 30 seconds to 20 minutes.

5. A method of any one of claims 1 to 4 wherein the pH of the lactose-containing liquid stream is adjusted or maintained to a pH of 6.7 to 8.0.

6. A method of any one of claims 3 to 5 wherein centrifugal separation or ultrafiltration of the pH adjusted, heated, or pH adjusted and heated lactose-containing liquid stream removes at least 60 to 95% by weight of the calcium from the lactose-containing liquid stream.

7. A method of any one of claims 1 to 6 wherein a fraction comprising 40 to 100% of the light phase from the centrifugal separator is recycled to the lactose-containing liquid stream feeding into the centrifugal separator.

8. A method of any one of claims 1 to 7 wherein the lactose-containing liquid stream feeding into the centrifugal separator or filtration is diluted by addition of water.

9. A method of claim 7 wherein the calcium concentration in the lactose-containing liquid stream feeding to the centrifugal separator or filtration is diluted to 0.2 to 0.7% by weight.

10. A method of any one of claims 1 to 9 wherein the lactose-containing liquid stream is selected from
i) mother liquor,
ii) wash water, or
iii) mother liquor and wash water.

11. A method of claims 1 to 10 wherein:
the ultrafiltration membrane is a high-temperature tolerant membrane; or
the molecular weight cut-off (MWCO) of the ultrafiltration membrane is 1,000 Daltons to 10,000 Daltons;
and temperature of the ultrafiltration feed stream is up to 80° C to 90° C.

12. A method of any one of claims 1 to 11 wherein the temperature of the nanofiltration feed stream is 45° C to 90° C.

13. A method of any one of claims 1 to 12 wherein diafiltration is carried out with water and HCl having a concentration of 0.0625% w/w HCl.

14. A method of any one of claims 1 to 13 wherein the pH of the nanofiltration feed stream is adjusted to 5.8 to 6.2.

15. A method of any one of claim 1 to 14 wherein the lactose-containing extract comprises at least 70% by weight to 85% by weight lactose on a dry basis.

16. A method of any one of claims 1 to 15 wherein the method of recovering lactose results in a lactose yield from the lactose-containing liquid source of at least 70% to 95%.

## Patentansprüche

1. Verfahren zum Erhalten von Lactose aus einer Lactose enthaltenden Flüssigkeitsquelle, umfassend:
Unterziehen einer Lactose enthaltenden Flüssigkeitsquelle einem Lactose-Kristallisationsverfahren, um einen oder mehrere Lactose enthaltende Flüssigkeitsströme zu erzeugen,
Unterziehen des einen oder der mehreren Lactose enthaltenden Flüssigkeitsströme (i) Wärme, (ii) einer Einstellung des pH-Werts oder (iii) Wärme und einer Einstellung des pH-Werts,
Unterziehen des einen oder der mehreren Lactose enthaltenden Flüssigkeitsströme einem oder mehreren Verfahren zur Erzeugung eines Lactose enthaltenden Extrakts, wobei das eine oder die mehreren Verfahren umfassen
i) einen ersten Schritt, um Zellen, Proteine, Polypeptide, Polysaccharide, Lipide, Ionen und/oder Salze zu entfernen, umfassend
a) Zentrifugaltrennung zur Erzeugung einer leichten Phase und einer schweren Phase mit optionaler Ultrafiltration oder Nanofiltration der leichten Phase oder
b) Ultrafiltration zur Erzeugung eines Ultrafiltrationspermeats und eines Ultrafiltrationsretentats und
ii) einen zweiten Schritt, umfassend eine Nanofiltration
a) der leichten Phase aus dem Zentrifugalabscheider oder
b) des Permeats aus der Ultrafiltration oder
c) des Retentats aus der Nanofiltration,
wobei mindestens ein Teil des Lactose enthaltenden Extrakts der Lactose enthaltenden Flüssigkeitsquelle vor, während oder nach der Eintragung in den Kristallisationsprozess zugesetzt wird.

2. Verfahren nach Anspruch 1, wobei der Lactose enthaltende Flüssigkeitsstrom vor dem Verarbeiten durch Zentrifugation oder Filtration
i) Erwärmen auf eine erste Temperatur, oder
ii) Einstellen des pH-Werts oder
iii) Erwärmen auf eine erste Temperatur und Einstellen des pH-Werts oder
iv) Einstellen des pH-Werts und Erwärmen auf eine erste Temperatur
unterzogen wird.

3. Verfahren nach Anspruch 2, wobei der Lactose enthaltende Flüssigkeitsstrom auf eine erste Temperatur von 70 °C bis 100 °C erwärmt wird.

4. Verfahren nach Anspruch 3, wobei der Lactose enthaltende Flüssigkeitsstrom 30 Sekunden bis 20 Minuten lang auf der erwärmten Temperatur gehalten wird.

5. Verfahren nach einem der Ansprüche 1 bis 4, wobei der pH-Wert des Lactose enthaltenden Flüssigkeitsstroms auf einen pH-Wert von 6,7 bis 8,0 eingestellt oder bei einem pH-Wert von 6,7 bis 8,0 gehalten wird.

6. Verfahren nach einem der Ansprüche 3 bis 5, wobei Zentrifugaltrennung oder Ultrafiltration des pH-eingestellten, erwärmten oder pH-eingestellten und erwärmten Lactose enthaltenden Flüssigkeitsstroms mindestens 60 bis 95 Gew.-% des Calciums aus dem Lactose enthaltenden Flüssigkeitsstrom entfernt.

7. Verfahren nach einem der Ansprüche 1 bis 6, wobei eine Fraktion umfassend 40 bis 100 % der leichten Phase aus dem Zentrifugalabscheider in den Lactose enthaltenden Flüssigkeitsstrom, der dem Zentrifugalabscheider zugeführt wird, zurückgeführt wird.

8. Verfahren nach einem der Ansprüche 1 bis 7, wobei der Lactose enthaltende Flüssigkeitsstrom, der dem Zentrifugalabscheider oder der Filtration zugeführt wird, durch Zugabe von Wasser verdünnt wird.

9. Verfahren nach Anspruch 7, wobei die Calciumkonzentration in dem Lactose enthaltenden Flüssigkeitsstrom, der dem Zentrifugalabscheider oder der Filtration zugeführt wird, auf 0,2 bis 0,7 Gew.-% verdünnt wird.

10. Verfahren nach einem der Ansprüche 1 bis 9, wobei der Lactose enthaltende Flüssigkeitsstrom ausgewählt ist aus
i) Mutterlauge,
ii) Waschwasser oder
iii) Mutterlauge und Waschwasser.

11. Verfahren nach Anspruch 1 bis 10, wobei:
die Ultrafiltrationsmembran eine hochtemperaturtolerante Membran ist; oder
der Molekulargewichts-Cutoff (MWCO) der Ultrafiltrationsmembran 1.000 Dalton bis 10.000 Dalton beträgt;
und die Temperatur des Ultrafiltrationszufuhrstroms bis zu 80 °C bis 90 °C beträgt.

12. Verfahren nach einem der Ansprüche 1 bis 11, wobei die Temperatur des Nanofiltrationszufuhrstroms 45 °C bis 90 °C beträgt.

13. Verfahren nach einem der Ansprüche 1 bis 12, wobei eine Diafiltration mit Wasser und HCl mit einer Konzentration von 0,0625 Gew.-% HCl durchgeführt wird.

14. Verfahren nach einem der Ansprüche 1 bis 13, wobei der pH-Wert des Nanofiltrationszufuhrstroms auf 5,8 bis 6,2 eingestellt wird.

15. Verfahren nach einem der Ansprüche 1 bis 14, wobei der Lactose enthaltende Extrakt mindestens 70 Gew.-% bis 85 Gew.-% Lactose auf Trockenbasis umfasst.

16. Verfahren nach einem der Ansprüche 1 bis 15, wobei das Verfahren der Wiedergewinnung von Lactose zu einer Lactose-Ausbeute aus der Lactose enthaltenden Flüssigkeitsquelle von mindestens 70 % bis 95 % führt.

## Revendications

1. Procédé d'obtention de lactose à partir d'une source liquide contenant du lactose comprenant :
la soumission d'une source liquide contenant du lactose à un processus de cristallisation du lactose pour générer un ou plusieurs courants liquides contenant du lactose,
la soumission du ou des courants liquides contenant du lactose à (i) de la chaleur, (ii) un ajustement du PH, ou (iii) de la chaleur et un ajustement du pH,
la soumission du ou des courants liquides contenant du lactose à un ou plusieurs procédés pour générer un extrait contenant du lactose, le ou les procédés comprenant
i) une première étape, pour éliminer un ou plusieurs parmi des cellules, protéines, polypeptides, polysaccharides, lipides, ions ou sels, comprenant
a) la séparation centrifuge pour produire une phase légère et une phase lourde avec ultrafiltration ou nanofiltration facultative de la phase légère, ou
b) l'ultrafiltration pour générer un perméat d'ultrafiltration et un rétentat d'ultrafiltration, et
ii) une deuxième étape comprenant la nanofiltration de
a) la phase légère provenant du séparateur centrifuge, ou
b) le perméat de l'ultrafiltration, ou
c) le rétentat de la nanofiltration,
l'ajout au moins d'une partie de l'extrait contenant du lactose à la source liquide contenant du lactose avant, pendant ou après l'entrée dans le processus de cristallisation.

2. Procédé selon la revendication 1, le courant liquide contenant du lactose étant soumis au
i) chauffage à une première température, ou
ii) ajustement du PH, ou
iii) chauffage à une première température et ajustement du pH, ou
iv) ajustement du PH et chauffage à une première température, avant traitement par centrifugation ou filtration.

3. Procédé selon la revendication 2, le courant liquide contenant du lactose étant chauffé à une première température de 70 °C à 100 °C.

4. Procédé selon la revendication 3, le courant liquide contenant du lactose étant maintenu à la température chauffée pendant 30 secondes à 20 minutes.

5. Procédé selon l'une quelconque des revendications 1 à 4, le pH du courant liquide contenant du lactose étant ajusté ou maintenu à un pH de 6,7 à 8,0.

6. Procédé selon l'une quelconque des revendications 3 à 5, la séparation centrifuge ou l'ultrafiltration du courant liquide contenant du lactose, ajusté en pH, chauffé, ou ajusté en pH et chauffé, éliminant au moins 60 à 95 % en poids du calcium du courant liquide contenant du lactose.

7. Procédé selon l'une quelconque des revendications 1 à 6, une fraction comprenant 40 à 100 % de la phase légère provenant du séparateur centrifuge étant recyclée dans le courant liquide contenant du lactose, qui alimente le séparateur centrifuge.

8. Procédé selon l'une quelconque des revendications 1 à 7, le courant liquide contenant du lactose, qui alimente le séparateur centrifuge ou la filtration, étant dilué par addition d'eau.

9. Procédé selon la revendication 7, la concentration de calcium dans le courant liquide contenant du lactose, qui alimente le séparateur centrifuge ou la filtration, étant diluée à 0,2 à 0,7 % en poids.

10. Procédé selon l'une quelconque des revendications 1 à 9, le courant liquide contenant du lactose étant choisi parmi
i) une liqueur mère,
ii) de l'eau de lavage, ou
iii) une liqueur mère et de l'eau de lavage.

11. Procédé selon les revendications 1 à 10,
la membrane d'ultrafiltration étant une membrane tolérante aux hautes températures ; ou
le seuil de coupure du poids moléculaire (MWCO) de la membrane d'ultrafiltration étant de 1000 Daltons à 10000 Daltons ;
et la température du courant d'alimentation de l'ultrafiltration pouvant atteindre 80 °C à 90 °C.

12. Procédé selon l'une quelconque des revendications 1 à 11, la température du courant d'alimentation de nanofiltration étant de 45 °C à 90 °C.

13. Procédé selon l'une quelconque des revendications 1 à 12, la diafiltration étant effectuée avec de l'eau et du HCl ayant une concentration de 0,0625 % p/p de HCl.

14. Procédé selon l'une quelconque des revendications 1 à 13, le pH du courant d'alimentation de nanofiltration étant ajusté entre 5,8 et 6,2.

15. Procédé selon l'une quelconque des revendications 1 à 14, l'extrait contenant du lactose comprenant au moins 70 % en poids à 85 % en poids de lactose sur une base sèche.

16. Procédé selon l'une quelconque des revendications 1 à 15, le procédé de récupération du lactose résultant en un rendement en lactose à partir de la source liquide contenant du lactose d'au moins 70 à 95 %.
